# EUROPÄISCHE PATENTSCHRIFT

(11) **EP 1 866 150 B1**
(45) Veröffentlichungstag und Bekanntmachung des Hinweises auf die Patenterteilung: **19.10.2016**
(21) Anmeldenummer: 06725349.2
(22) Anmeldetag: 28.03.2006
(51) Int. Cl.: C08J 7/04, C09J 189/00, C09D 189/00, A61L 24/10, A61L 27/34, B05D 3/10

(54) **METALLISCHE SUBSTRATE MIT POLYPEPTIDEN ALS HAFTVERMITTLER**
METALLIC SUBSTRATES HAVING POLYPEPTIDES AS ADHESIVE AGENTS
SUBSTRATS MÉTALLIQUES AYANT DES POLYPEPTIDES COMME AGENTS ADHESIFS

(30) Priorität: 31.03.2005 DE 102005015043; 23.09.2005 DE 102005045770
(43) Veröffentlichungstag der Anmeldung: 19.12.2007
(73) Patentinhaber: BASF SE, 67056 Ludwigshafen am Rhein (DE)
(72) Erfinder: SEYFFER, Hermann, 69123 Heidelberg (DE); SCHUMACHER, Karl-Heinz, 67433 Neustadt (DE); LEMAIRE, Hans-Georg, 67117 Limburgerhof (DE); BAUS, Ulf, 69221 Dossenheim (DE); SUBKOWSKI, Thomas, 68526 Ladenburg (DE); KAROS, Marvin, 68723 Schwetzingen (DE); BOLLSCHWEILER, Claus, 69118 Heidelberg (DE); HEIDENFELDER, Thomas, 67125 Dannstadt-Schauernheim (DE)
(74) Vertreter: BASF IP Association
(86) Internationale Anmeldenummer: PCT/EP2006/061085
(87) Internationale Veröffentlichungsnummer: WO 2006/103225

(56) Entgegenhaltungen:
- WO-A-03/018673
- WO-A-03/080137
- WO-A-2004/000880
- US-A- 2 399 161
- US-A- 3 751 280
- US-A- 5 015 677
- US-A- 5 049 504
- US-A- 5 859 198
- US-A1- 2003 134 042
- HIDER G C: "A RELATIVELY SIMPLE TEST FOR THE DIRECT DETERMINATION OF THE CYSTEINE CONTENT IN PHOTOGRAPHIC GELATIN USING A THIOL-SPECIFIC FLUOROGENIC REAGENT" IMAGING SCIENCE JOURNAL, THE, MANEY PUBLISHING, LONDON, GB, Bd. 45, Nr. 3/4, 1997, Seiten 162-166, XP000752031 ISSN: 1368-2199
- PATENT ABSTRACTS OF JAPAN Bd. 1996, Nr. 03, 29. März 1996 (1996-03-29) & JP 07 289261 A (KAIYO BIO TECHNOL KENKYUSHO:KK), 7. November 1995 (1995-11-07)
- PATENT ABSTRACTS OF JAPAN Bd. 1997, Nr. 02, 28. Februar 1997 (1997-02-28) & JP 08 266281 A (KAIYO BIO TECHNOL KENKYUSHO:KK), 15. Oktober 1996 (1996-10-15)

## Beschreibung

Die Erfindung betrifft ein beschichtetes Substrat, enthaltend Verbindungen, welche zu mindestens 40 Gew.-% aus über Peptidbindungen verknüpften alpha-Aminosäuren aufgebaut sind (im nachfolgenden kurz Polypeptid genannt), als Haftvermittler zwischen der Beschichtung und dem Substrat. Insbesondere betrifft die Erfindung die Verwendung von Hydrophobinen als Haftvermittler.

Hydrophobine sind kleine Proteine von etwa 100 bis 150 Aminosäuren, die charakteristisch für filamentöse Pilze, beispielsweise Schizophyllum commune, sind. Sie weisen in aller Regel 8 Cystein-Einheiten auf.

Hydrophobine weisen eine ausgeprägte Affinität zu Grenzflächen auf und eignen sich daher zur Beschichtung von Oberflächen. So lässt sich beispielsweise Teflon mittels Hydrophobinen unter Erhalt einer hydrophilen Oberfläche beschichten.

Hydrophobine können aus natürlichen Quellen isoliert werden. Unsere ältere Anmeldung DE 10 2005 007 480.4 offenbart ein Herstellverfahren für Hydrophobine.

Im Stand der Technik ist die Verwendung von Hydrophobinen für verschiedene Anwendungen vorgeschlagen worden.

WO 96/41882 schlägt die Verwendung von Hydrophobinen als Emulgataren, Verdicker, oberflächenaktive Substanzen, zum Hydrophilieren hydrophober Oberflächen, zur Verbesserung der Wasserbeständigkeit hydrophiler Substrate, zur Herstellung von Öl-in-Wasser-Emulsionen oder von Wasser-in-Öl-Emulsionen vor. Weiterhin werden pharmazeutische Anwendungen wie die Herstellung von Salben oder Cremes sowie kosmetische Anwendungen wie Hautschutz oder die Herstellung von Haarshampoos oder Haarspülungen vorgeschlagen.

EP 1 252 516 offenbart die Beschichtung von Fenstern, Kontaktlinsen, Biosensoren, medizinischen Vorrichtungen, Behältern zur Durchführung von Versuchen oder zur Lagerung, Schiffrümpfen, festen Teilchen oder Rahmen oder Karosserie von Personenkraftwagen mit einer Hydrophobine enthaltenden Lösung bei einer Temperatur von 30 bis 80°C.

WO 03/53383 offenbart die Verwendung von Hydrophobin zum Behandeln von Keratin-Materialien in kosmetischen Anwendungen,

WO 03/10331 offenbart einen mit Hydrophobin beschichteten Sensor, beispielsweise eine Messelektrode, an den nicht kovalent weitere Substanzen, z. B. elektroaktive Substanzen, Antikörper oder Enzyme gebunden sind.

US 5015677 betrifft einen Klebstoff oder eine Beschichtungszusammensetzung für biomedizinische Anwendungen enthaltend ein polyphenolisches Protein mit einer bestimmten aus Wiederholungseinheiten aufgebauten Struktur, ein Additiv und einen Füllstoff.

US 2003/134042 betrifft die Behandlung eines Objektes, das eine Fensterscheibe, eine Kontaktlinse, ein Biosensor. ein medizinisches Gerät, ein Versuchs- oder Aufbewahrungsbehälter, ein Schiffskörper oder ein Fahrzeugchassis ist, mit einer Hydrophobin-haltigen Lösung zur Beschichtung dieses Objekts mit Hydrophobin.

Bisher werden unterschiedlichste Haftvermittler zur Verbesserung der Haftfestigkeit, z. B. von Beschichtungen auf unterschiedlichsten Substraten verwendet. Gemäß Römpp Chemie Lexikon (Auflage 1990) kommen z. B. Titanate, Silane, Chrom-Komplexe ungesättigter Carbonsäuren, in Betracht. Speziell als Haftvermittler für Klebstoffe werden Ethylen/Acylamid Copolymere, polymere Isocyanate oder reaktive Silicium-organische Verbindungen genannt.

Bekannt sind auch Polyurethane und Polyethylenimine als Haftvermittler.

Aufgabe der vorliegenden Erfindung war es, alternative Haftvermittler bereitzustellen, welche möglichst gute anwendungstechnische Eigenschaften haben und insbesondere eine gute Haftung der einzelnen Schichten eines mehrschichtigen Verbunds oder einer Beschichtung auf einem Substrat bewirkt.

Demgemäß wurde das beschichtete Substrat gefunden.

### Zum Haftvermittler

Der mehrschichtige Verbund oder das beschichtete Substrat enthält das eingangs definierte Polypeptid als Haftvermittler.

Das Polypeptid besteht zu mindestens 40 Gen.-%, vorzugsweise zu mindestens 70 Gew.-%, besonders bevorzugt zu mindestens 90 Gew.-% und ganz besonders bevorzugt zu mindestens 95 oder 99 Gew.-% aus über Peptidbindungen verknüpften alpha-Aminosäuren.

In einer besonderen Ausführungsform besteht das Polypeptid ausschließlich aus über Peptidbindungen verknüpften alpha-Aminosäuren.

Als alpha-Aminosäuren in Betracht kommen insbesondere Glycin, Alanin,Valin, Leucin, Isoleucin, Phenylalanin, Tyrosin, Prolin, Hydroxyprolin, Serin, Threonin, Cystein, Cystin, Methionin, Tryptophan, Asparaginsäure, Glutaminsäure, Arginin, Lysin und Histidin.

Vorzugsweise enthält das Polypeptid Cystein als alpha-Aminosäure im Gemisch mit anderen alpha-Aminosäuren.

Besonders bevorzugt besteht das Polypeptid zu mindestens 0,1 Gew.-%, besonders bevorzugt zu mindestens 0,5, ganz besonders bevorzugt zu mindestens 1 Gew.-% aus Cystein. Der Gehalt an Cystein im Polypeptid übersteigt im allgemeinen nicht Werte von 15 Gew.-% insbesondere nicht Werte von 10 Gew.-% und ganz besonders bevorzugt übersteigt er nicht Werte von 7 Gew.-%.

In einer besonderen Ausführungsform handelt es sich bei den Polypeptiden um Hydrophobine.

Unter dem Begriff "Hydrophobine" im Sinne dieser Erfindung sollen im Folgenden Proteine der allgemeinen Strukturformel (I)

Xₙ-C¹-X₁₋₅₀-C²-X₀₋₅-C³-X₁₋₁₀₀-C⁴-X₁₋₁₀₀-C⁵-X₁₋₅₀-C⁶-X_{0.5}-C⁷-X₁₋₅₀-C⁸-Xₘ (I)

verstanden werden, wobei X für jede der 20 natürlich vorkommenden Aminosäuren (Phe, Leu, Ser, Tyr, Cys, Trp, Pro, His, Gln, Arg, Ile Met, Thr, Asn, Lys, Val, Ala, Asp, Glu, Gly) stehen kann. Dabei können X jeweils gleich oder verschieden sein. Hierbei stellen die bei X stehenden Indizes jeweils die Anzahl der Aminosäuren dar, C steht für Cystein, Alanin, Serin, Glycin, Methionin oder Threonin, wobei mindestens vier der mit C benannten Reste für Cystein stehen, und die Indizes n und m stehen unabhängig voneinander für natürliche Zahlen von 0 und 500, bevorzugt von 15 bis 300.

Die Polypetide gemäß Formel (I) sind weiterhin durch die Eigenschaft charakterisiert, dass sie bei Raumtemperatur nach Beschichten einer Glasoberfläche eine Vergrößerung des Kontaktwinkels eines Wassertropfens von mindestens 20°, bevorzugt mindestens 25° und besonders bevorzugt 30° bewirken, jeweils verglichen mit dem Kontaktwinkel eines gleich großen Wassertropfens mit der unbeschichteten Glasoberfläche.

Die mit C¹ bis C⁸ benannten Aminosäuren sind bevorzugt Cysteine; sie können aber auch durch andere Aminosäuren ähnlicher Raumerfüllung, bevorzugt durch Alanin, Serin, Threonin, Methionin oder Glycin ersetzt werden. Allerdings sollen mindestens vier, bevorzugt mindestens 5, besonders bevorzugt mindestens 6 und insbesondere mindestens 7 der Positionen C¹ bis C⁸ aus Cysteinen bestehen. Cysteine können in den erfndungsgemässen Proteinen entweder reduziert vorliegen oder miteinander Disulfidbrücken ausbilden. Besonders bevorzugt ist die intramolekulare Ausbildung von C-C Brücken, insbesondere die mit mindestens einer, bevorzugt 2, besonders bevorzugt 3 und ganz besonders bevorzugt 4 intramolekularen Disulfidbrücken. Bei dem oben beschriebenen Austausch von Cysteinen durch Aminosäuren ähnlicher Raumerfüllung werden vorteilhaft solche C-Positionen paarweise ausgetauscht, die intramolekulare Disulfidbrücken untereinander ausbilden können.

Falls in den mit X bezeichneten Positionen auch Cysteine, Serine, Alanine. Glycine, Methionine oder Threonine verwendet werden, kann sich die Nummerierung der einzelnen C-positionen in den allgemeinen Formeln entsprechend verändern.

Bevorzugt werden Hydrophobine der allgemeinen Formel (II)

Xₙ-C¹-X₃₋₂₅-C²-X₀₋₂-C³-X₅₋₅₀-C⁴-X₂₋₃₅-C⁵-X₂₋₁₅-C⁶-X₀₋₂-C⁷-X₃₋₃₅-C⁸-Xₘ (II)

zur Ausführung der vorliegenden Erfindung eingesetzt, wobei X, C und die bei X und C stehenden Indizes die obige Bedeutung haben, jedoch stehen die Indizes n und m für Zahlen zwischen 0 und 300, und sich die Proteine weiterhin durch die oben erwähnte Kontaktwinkeländerung auszeichnen.

Besonders bevorzugt werden Hydrophobine der allgemeinen Formel (III)

Xₙ-C¹-X₅₋₉-C²-C³-X₁₁₋₃₉-C⁴-X₂₋₂₃-C⁵-X₅₋₉-C⁶-C⁷-X₆₋₁₈-C⁸-Xₘ (III)

eingesetzt, wobei X, C und die bei X und C stehenden Indizes die obige Bedeutung haben, die Indizes n und m für Zahlen zwischen 0 und 200 stehen, und sich die Proteine weiterhin durch die oben erwähnte Kontaktwinkeländerung auszeichnen, und es sich weiterhin bei mindestens 6 der mit C benannten Reste um Cystein handelt. Besonders bevorzugt handelt es sich bei allen Reste C um Cystein.

Bei den Resten Xₙ und Xₘ kann es sich um Peptidsequenzen handeln, die natürlicherweise mit einem Hydrophobin verknüpft sind. Es kann sich aber auch bei einem oder beiden Resten um Peptidsequenzen handeln, die natürlicherweise nicht mit einem Hydrophobin verknüpft sind. Darunter sind auch solche Reste Xₙ und/oder Xₘ zu verstehen, bei denen eine natürlicherweise in einem Hydrophobin vorkommende Peptidsequenz durch eine nicht natürlicherweise in einem Hydrophobin vorkommende Peptidsequenz verlängert ist.

Falls es sich bei Xₙ und/oder Xₘ um natürlicherweise nicht mit Hydrophobinen verknüpfte Peptidsequenzen handelt, sind derartige Sequenzen in der Regel mindestens 20, bevorzugt mindestens 35, besonders bevorzugt mindestens 50 und ganz besonders bevorzugt mindestens 100 Aminosäuren lang. Ein derartiger, natürlicherweise nicht mit einem Hydrophobin verknüpfter Rest soll im Folgenden auch als Fusionspartner bezeichnet werden. Damit soll ausgedrückt werden, dass die Proteine aus mindestens einem Hydrophobinteil und einem Fusionspartner bestehen können, die in der Natur nicht zusammen in dieser Form vorkommen.

Der Fusionspartner kann aus einer Vielzahl von Proteinen ausgewählt werden. Es Können auch mehrere Fusionspartner mit einem Hydrophobinteil verknüpft werden, beispielsweise am Aminoterminus (Xₙ) und am Carboxyterminus (Xₘ) des Hydrophobinteils. Es können aber auch beispielsweise zwei Fusionspartnerteile mit einer Position (Xₙ oder Xₘ) des erfindungsgemäßen Proteins verknüpft werden.

Besonders geeignete Fusionspartnerteile sind Proteine, die natürlicherweise in Mikroorganismen, insbesondere in E. coli oder Bacillus subtilis vorkommen. Beispiele für solche Fusionspartnerteile sind die Sequenzen yaad (SEQ ID NO:15 und 16), yaae(SEQ ID NO:17 und 18), und Thioredoxin. Gut geeignet sind auch Fragmente oder Derivate dieser genannten Sequenzen, die nur einen Teil, bevorzugt 70-99%, besonders bevorzugt 80-98% der genannten Sequenzen umfassen, oder bei denen einzelne Aminosäuren, bzw. Nukleotide gegenüber der genannten Sequenz verändert sind, wobei sich die Prozentangaben jeweils auf die Anzahl der Aminosäuren bezieht.

Die erfindungsgemäß verwendeten Proteine können auch noch in ihrer Polypeptidsequenz modifiziert sein, beispielsweise durch Glycosilierung, Acetylierung oder auch durch chemische Quervernetzung beispielsweise mit Glutardialdehyd.

Eine Eigenschaft der erfindungsgemäß verwendeten Proteine ist die Änderung von Oberflächeneigenschaften, wenn die Oberflächen mit den Proteinen beschichtet werden. Die Änderung der Oberflächeneigenschaften lässt sich experimentell dadurch bestimmen, dass der Kontaktwinkel eines Wassertropfens vor und nach der Beschichtung der Oberfläche mit dem Protein gemessen wird und die Differenz der beiden Messungen ermittelt wird.

Die Durchführung von Kontaktwinkelmessungen ist dem Fachmann prinzipiell bekannt. Die Messungen beziehen sich auf Raumtemperatur sowie Wassertropfen von 5 I. Die genauen experimentellen Bedingungen für eine beispielhaft geeignete Methode zur Messung des Kontaktwinkel sind im experimentellen Teil dargestellt. Unter den dort genannten Bedingungen besitzen die erfingdungsgemäß verwendeten Proteine die Eigenschaft, den Kontaktwinkel um mindestens 20°, bevorzugt mindestens 25°, besonders bevorzugt mindestens 30° zu vergrößern, jeweils verglichen mit dem Kontaktwinkel eines gleich großen Wassertropfens mit der unbeschichteten Glasoberfläche.

Im Hydrophobinteil der bisher bekannten Hydrophobine sind die Positionen der polaren und unpolaren Aminosäuren konserviert, was sich in einem charakteristischen Hydrophobizitätsplot äußert. Unterschiede in den biophysikalischen Eigenschaften und in der Hydrophobizität führten zur Einteilung der bisher bekannten Hydrophobine in zwei Klassen, 1 und 11 (Wessels et al. 1994, Ann. Rev. Phytopathol., 32, 413-437).

Die assemblierten Membranen aus Klasse I Hydrophobinen sind hochgradig unlöslich (selbst gegenüber 1% Na-Dodecylsulfat (SDS) bei erhöhter Temperatur) und können nur durch konzentrierte Trifluoressigsäure (TFA), bzw. Ameisensäure wieder dissoziiert werden. Im Gegensatz dazu sind die assemblierten Formen von Klasse II Hydrophobinen weniger stabil. Sie können bereits durch 60%iges Ethanol, bzw. 1% SDS (bei Raumtemperatur) wieder aufgelöst werden.

Ein Vergleich der Aminosäuresequenzen zeigt, dass die Länge des Bereichs zwischen Cystein C³ und C⁴ bei Klasse II Hydrophobinen deutlich kürzer ist, als bei Hydrophobinen der Klasse I. Klasse II Hydrophobine weisen weiterhin mehr geladene Aminosäuren als Klasse I auf.

Besonders bevorzugte Hydrophobine zur Ausführung der vorliegenden Erfindung sind die Hydrophobine des Typs dewA, rodA, hypA, hypB, sc3, basf1, basf2, die im nachfolgenden Sequenzprotokoll strukturell charakterisiert sind. Es kann sich auch nur um Teile oder Derivate davon handeln. Es können auch mehrere Hydrophobinteile, bevorzugt 2 oder 3, gleicher oder unterschiedlicher Struktur miteinander verknüpft und mit einer entsprechenden geeigneten Polypeptidsequenz, die natürlicherweise nicht mit einem Hydrophobin verbunden ist, verknüpft werden.

Besonders geeignet zur Durchführung der vorliegenden Erfindung sind weiterhin die Fusionsproteine mit den in SEQ ID NO: 20, 22, 24 dargestellten Polypeplidsequenzen sowie den dafür codierenden Nukleinsäuresequenzen, insbesondere den Sequenzen gemäss SEQ ID NO: 19, 21, 23. Auch Proteine, die sich ausgehend von den in SEQ ID NO. 20, 22 oder 24 dargestellten Polypeptidsequenzen durch Austausch, Insertion oder Deletion von mindestens einer, bis hin zu 10. bevorzugt 5 , besonders bevorzugt 5% aller Aminosäuren ergeben, und die die biologische Eigenschaft der Ausgangsproteine noch zu mindestens 50% besitzen, sind besonders bevorzugte Ausführungsformen. Unter biologischer Eigenschaft der Proteine wird hierbei die bereits beschriebene Vergrößerung des Kontaktwinkels um mindestens 20° verstanden.

Die erfindungsgemäß verwendeten Proteine lassen sich chemisch durch bekannte Verfahren der Peptidsynthese, beispielsweise durch Festphasensynthese nach Merrifield herstellen.

Natürlich vorkommende Hydrophobine lassen sich aus natürlichen Quellen mittels geeigneter Methoden isolieren. Beispielhaft sei auf Wösten et. al., Eur. J Cell Bio- 63, 122-129 (1994) oder WO 96/41882 verwiesen.

Die Herstellung von Fusionsproteinen kann bevorzugt durch gentechnische Verfahren erfolgen, bei denen eine für den Fusionspartner und eine für den Hydrophobinteil codierende Nukleinsäuresequenz, insbesondere DNA-Sequenz, so kombiniert werden, dass in einem Wirtsorganismus durch Genexpression der kombinierten Nukleinsäuresequenz das gewünschte Protein erzeugt wird. Ein derartiges Herstellverfahren ist in unserer älteren Anmeldung DE 102005007480.4 offenbart.

Geeignete Wirtsorganismen (Produktionsorganismen) für das genannte Herstellverfahren können dabei Prokaryonten (einschließlich der Archaea) oder Eukaryonten sein, besonders Bakterien einschliesslich Halobacterien und Methanococcen, Pilze, Insektenzellen, Pflanzenzellen und Säugerzellen, besonders bevorzugt Escherichia coli, Bacillus subtilis, Bacillus, megaterium, Aspergillus oryzea, Aspergillus nidulans, Aspergillus niger, Pichia pastoris, Pseudomonas spec., Lactobacillen, Hansenula polymorpha, Trichoderma reesei, SF9 (bzw. verwandte Zellen) u.a.

Gegenstand der Erfindung ist außerdem die Verwendung von Expressionskonstrukten, enthaltend unter der genetischen Kontrolle regulativer Nukleinsäuresequenzen, eine für ein erfindungsgemäß verwendetes Polypeptid kodierende Nukleinsäuresequenz, sowie Vektoren, umfassend wenigstens eines dieser Expressionskonstrukte.

Vorzugsweise umfassen eingesetzte Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann.

Beispiele für operativ verknüpfbare Sequenzen sind Targeting-Sequenzen sowie Einhancer, Polyadenylierungssignale und dergleichen. Weitere regulative Elemente umfassen selektierbare Marker. Amplifikationssignale, Replikationsursprünge und dergleichen. Geeignete regulatorische Sequenzen sind z. B. beschrieben in Goeddel, Gene Expression Techno- logy: Methods in Enzymology 185. Academic Press, San Diego, CA(1990).

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde.

Ein bevorzugtes Nukleinsäurekonstrukt enthält vorteilhaft auch eine oder mehrere der schon erwähnten "Enhancer"-Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder rerminataren.

Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das Verfahren sind beispielsweise in Promotoren wie cos-, tac-, trp-, tet-, trp-tet-, Ipp-, Iac-,Ipp-Iac-, IacIq-T7- , T5-, T3-, gaI-, trc-, ara-, rhaP(rhaPBAD) SP6-, lambda-PR-oder imlambda-P-Promotor enthalten, die vorteilhaft in gram-negativen Bakterien Anwendung finden. Weitere vorteilhafte Regulationssequenzen sind beispielsweise in den gram-positiven Promotoren amy und SP02, in den Hefe-oder Pilzpromotoren ADC1, MFalpha, AC, P-60, CYC1, GAPDH, TEF, rp28, ADH enthalten.

Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z. B. Viren, wie SV40, CMV, Baculovirus und Adenovirus, Transposons,IS- Elemente, Phasmide, Cosmide, und lineare oder zirkuläre DNA, sowie das Agrobacterium-System zu verstehen.

Diese Vektoren können autonom im Wirtsorganismus repliziert oder chromosomal repliziert werden. Diese Vektoren stellen eine weitere Ausgestaltung der Erfindung dar Geeignete Plasmide sind beispielsweise in E. coli pLG338, pACYC184, pBR322, pUC18,pUC19, pKC30, pRep4, pHS1. pKK223-3, pDHE19.2, pHS2, pPLc236, pMBL24, pLG200, pUR290,pIN-III"3-B1, tgt11 oder pBdCI, in StreptomycespIJ101, pIJ364,pIJ702 oderpIJ361, in Bacillus pUB110, C194 oder pBD214, in Corynebacterium pSA77 oder pAJ667, in Pilzen pALS1, pIL2 oder pBB116, in Hefen 2alpha, pAG-1, YEp6, YEp13 oder pEMBLYe23 oder in Pflanzen pLGV23,pGHIac+, pBIN19, pAK2004 oder pDH51. Die genannten Plasmide stellen eine kleine Auswahl der möglichen Plasmide dar. Weitere Plasmide sind dem Fachmann bekannt und können beispielsweise aus dem Buch Cloning Vectors (Eds. Pouwels P. H. et al. Elsevier, Ams- terdam-New York-Oxford, 1985, ISBN 0 444 904018) entnommen werden.

Vorteilhaft enthält das Nukleinsäurekonstrukt zur Expression der weiteren enthaltenen Gene zusätzlich noch 3'-und/oder 5'-terminale regutatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhaft in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft die Nukleinsäuresequenzen entsprechend des im Organismus verwendeten spezifischen "codon usage"zu verändern. Der "codon usage" lässt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E. F.Fritsch und J. Sambrook, Molecular Cloning : A Laboratory Manual, Cold Spring Harbor Laboratory, Cold Spring Harbor, NY (1989) sowie in T. J. Silhavy, M. L. Berman und L. W. Enquist. Experiments with Gene Fusions, Cold Spring Harbor Laboratory, Cold Spring Harbor, NY (1984) und in Ausubel, F. M. etal., Current Protocols in Molecular Biology, Greene Publishing Assoc. andwiley Interscience (1987) beschrieben sind.

Das rekombinante Nukleinsäurekonstrukt bzw. Genkonstrukt wird zur Expression in einem geeigneten Wirtsorganismus, vorteilhaft in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen wer- den.

Mit Hilfe der Vektoren sind rekombinante Mikroorganismen herstellbar, welche beispielsweise mit wenigstens einem Vektor transformiert sind und zur Produktion der erfindungsgemäß verwendeten Proteine eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F.Ausubel etal., Hrsg., Wiley Interscience, New York 1997, oder Sambrook et al. Molecular Claning : A Laboratory Manual. 2. Aufl., Cold Spring Harbor Laboratory, Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines erfingdungsgemäß zu verwendenden Gens oder einer kodierenden Sequenz enthält, worin gegebenenfalls wenigstens eine Aminosäure-Deletion, -Addition oder -Substitution eingebracht worden ist, um die Sequenz zu verändern, z. B. funktionell zu disruptieren ("Knockout"- Vektor). Die eingebrachte Sequenz kann z. B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, dass das endogene Gen bei homologer Rekombination mutiert oder anderweitig verändert ist, jedoch noch das funktionelle Protein kodiert (z. B. kann der stromaufwärts gelegene regulatorische Bereich derart verändert sein, dass dadurch die Expression des endogenen Proteins verändert wird). Der veränderte Abschnitt des erfindungsgemäß verwendeten Gens ist im homologen Rekombinationsvektor. Die Konstruktion geeigneter Vektoren zur homologen Rekombination ist z. B. beschrieben in Thomas, K. R. und Capecchi, M. R. (1987) Cell 51 : 503.

Als rekombinante Wirtsorganismen für die erfindungsgemäß verwendete Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden grampositive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet.

Die im Herstellverfahren für Fusionsproteine verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan- und Magnesiumsalze sowie gegebenenfalls Vitamine enthält, bei Temperaturen zwischen 0 und100 °C, bevorzugt zwischen 10 bis 60 °C unter Sauerstoffbegasung angezogen. Dabei kann der pH-Wert der Nährflüssigkeit auf einem festen Wert gehalten werden, das heißt während der Anzucht reguliert werden oder nicht. Die Anzucht kann "batch"-weise, "semi-batch"-weise oder kontinuierlich erfolgen. Nährstoffe können zu Beginn der Fermentation vorgelegt oder semikontinuierlich oder kontinuierlich nachgefüttert werden. Die Enzyme können nach dem in den Beispielen beschriebenen Verfahren aus den Organismen isoliert werden oder als Rohextrakt für die Reaktion verwendet werden.

Erfingdungsgemäß verwendete Proteine oder funktionelle, biologisch aktive Fragmente davon, können mittels eines rekombinanten Verfahrens hergestellt werden, bei dem man einen Proteine-produzierenden Mikroorganismus kultiviert, gegebenenfalls die Expression der Proteine induziert und diese aus der Kultur isoliert. Die Proteine können so auch in großtechnischem Maßstab produziert werden, falls dies erwünscht ist. Der rekombinante Mikroorganismus kann nach bekannten Verfahren kultiviert und fermentiert werden. Bakterien können beispielsweise in TB-oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E. F. Fritsch and J. Sambrook, Molecular Cloning A Laboratory Manual. Cold Spring Harbor Laboratory, Cold Spring Harbor, NY (1989) beschrieben

Die Zellen werden dann, falls die erfindungsgemäß verwerdeten Proteine nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der erfindunsgemäß verwendeten Proteine kann mit bekannten, chromatographischen Verfah- ren erzielt worden, wie Molekularsieb-Chromatographie (Gelfiltration), wie Q- Sepharose-Chromatographie, ionenaustausch-Chromatographie und hydrophobe Chromatographie, sowie mit anderen üblichen Verfahren wie Ultrafiltration, Kristallisati- on, Aussalzen, Dialyse und nativerGelelektrophorese. Geeignete Verfahren werden beispielsweise in Cooper, F. G., Biochemische Arbeitsmethoden, Verlag Water de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Proteine oder Fusionsproteine kodieren, die beispielsweise einer einfacheren Reinigung dienen. Derartige geeignete Modifikationen umfassen als Anker fungierende sogenannte "Tags", wie beispielsweise die als HexaHistidin-Anker bekannte Modifikation oder Epitope, die als Antigene von Antikörpern erkannt werden können (beschrieben zum Beispiel in Harlow, E. and Lane, D., 1988, Antibodies : A Laboratory Manual. Cold Spring Harbor (N. Y.) Press). Weitere geeignete Tags sind z.B. HA, Calmodulin-BD, GST, MBD; Chitin-BD, Steptavidin-BD-Avi-Tag, Flag-Tag, T7 etc. Diese Anker können zur Anheftung der Proteine an einen festen Träger, wie z. B. einer Polymermatrix, dienen, die beispielsweise in einer Chromatographiesäule eingefüllt sein kann, oder an einer Mikrotiterplatte oder an einem sonstigen Träger verwendet werden kann. Die entsprechenden Reinigungsprotokolle sind von den kommerziellen Affinitäts-Tag-Anbietern erhältlich.

Die wie beschrieben hergestellten Proteine können sowohl direkt als Fusionsproteine als auch nach Abspaltung und Abtrennung des Fusionspartners als "reine" Hydrophobine verwendet werden.

Wenn eine Abtrennung des Fusionspartners vorgesehen ist, empfiehlt es sich eine potentielle Spaltstelle (spezifische Erkennungsstelle für Proteasen) in das Fusionsprotein zwischen Hydrophobinteil und Fusionspartnerteil einzubauen. Als Spaltstelle geeignet sind insbesondere solche Peptidsequenzen geeignet, die ansonsten weder im Hydrophobinteil noch im Fusionspartnerteil vorkommen, was sich mit bioinformatischen Tools leicht ermitteln lässt. Besonders geeignet sind beispielsweise BrCN-Spaltung an Methionin, oder durch Protease vermittelte Spaltlung mit Faktor Xa-, Enterokinase-, Thrombin, TEV-Spaltung (Tobacca etch virus Protease).

Zuordnung der Sequenznamen zu DNA- und Polypeptidsequenzen im Sequenzprotokoll

| | |
|---|---|
| dewA DNA- und Polypeptidsequenz | SEQ ID NO: 1 |
| dewA Polypeptidsequenz | SEQ ID NO: 2 |
| rodA DNA- und Polypeptidsequenz | SEQ ID NO: 3 |
| rodA Polypeptidsequenz | SEQ ID NO: 4 |
| hypA DNA- und Polypeptidsequenz | SEQ ID NO: 5 |
| hypA Polypeptidsequenz | SEQ ID NO: 6 |
| hypB DNA- und Polypeptidsequenz | SEQ ID NO: 7 |
| hypB Polypeptidsequenz | SEQ ID NO: 8 |
| sc3 DNA- und Polypeptidsequenz | SEQ ID NO: 9 |
| sc3 Polypeptidsequenz | SEQ ID NO: 10 |
| basf1 DNA- und Polypeptidsequenz | SEQ ID NO: 11 |
| basf1 Polypeptidsequenz | SEQ ID NO: 12 |
| basf2 DNA- und Polypeptidsequenz | SEQ ID NO: 13 |
| basf2 Polypeptidsequenz | SEQ ID NO: 14 |
| yaad DNA- und Polypeptidsequenz | SEQ ID NO: 15 |
| yaad Polypeptidsequenz | SEQ ID NO: 16 |
| yaae DNA- und Polypeptidsequenz | SEQ ID NO: 17 |
| yaae Polypeptidsequenz | SEQ ID NO: 18 |
| yaad-Xa-dewA-his DNA- und Polypeptidsequenz | SEQ ID NO: 19 |
| yaad-Xa-dewA-his Polypeptidsequenz | SEQ ID NO: 20 |
| yaad-Xa-rodA-his DNA- und Polypeptidsequenz | SEQ ID NO: 21 |
| yaad-Xa-rodA-his Polypeptidsequenz | SEQ ID NO: 22 |
| yaad-Xa-basf1-his DNA- und Polypeptidsequenz | SEQ ID NO: 23 |
| yaad-Xa-basf1-his Polypeptidsequenz | SEQ ID NO: 24 |

### Zum mehrschichtigen Verbund (der mehrschichtige Verbund ist nicht erfindungsgemäß)

Mehrschichtige Verbunde werden für unterschiedlichste Zwecke, z. B. als Verpackungsmittel (insbesondere Verbundfolien) oder selbstklebende Artikel (mehrschichtiger Verbund aus mindestens Träger und Klebstoffschicht) eingesetzt.

Die mehrschichtigen Verbunde enthalten mindestens zwei, vorzugsweise zwei bis fünf Schichten, wobei die einzelnen Schichten eine Dicke von z. B. 0,01 bis 5 mm haben können. Die einzelnen Schichten können aus natürlichen oder synthetischen Polymeren oder auch aus Metall bestehen. Bei den Schichten handelt es sich insbesondere um Polymerfolien, Papier, Metallfolien, metallisierte Polymerfolien etc.

Zwischen mindestens zwei benachbarten Schichten des mehrschichtigen Verbunds werden die obigen Polypeptide als Haftvermittler verwendet. Vorzugsweise handelt es sich bei mindestens einer der benachbarten Schichten um eine Schicht aus einem natürlichen oder synthetischen Polymeren. Als Polymere kommen insbesondere Polykondensate, wie Polyester, Polyaddukte wie Polyurethane, Polyamide, Polycarbonate oder Polyphenylenether oder Polyphenylensulfide oder Polymere, welche durch radikalische oder ionische Polymerisation von ethylenisch ungesättigten Verbindungen erhältlich sind (kurz radikalische Polymere genannt). Derartige radikalische Polymere bestehen vorzugsweise zu mindestens 60 Gew.-%, besonders bevorzugt zu mindestens 80 Gew.-% aus sogenannten Hauptmonomeren, ausgewählt aus C1 bis C20 Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C Atome enthaltenden Alkoholen, aliphatischen Kohlenwasserstoffen mit 2 bis 8 C Atomen und ein oder zwei Doppelbindungen, insbesondere Ethylen und Propylen.

Die erfindungsgemäßen Polypeptide eignen sich insbesondere auch als Haftvermittler für unpolare Polymere; daher besteht vorzugsweise zumindest eine der benachbarten Schichten aus einem unpolaren Polymer.

Ein Maß für die Polarität von Polymeren ist die Oberflächenspannung gegenüber Luft (21°C). Je geringer die Oberflächenspannung, desto unpolarer ist das Polymer.

Vorzugsweise besteht daher zumindset eine der benachbarten Schichten aus einem unpolaren Polymer mit einer Oberflächenspannung kleiner 50 mN/m (milli-Newton/Meter), insbesondere kleiner 40 mN/m. Derartige unpolare Polymere sind z. B. Polyamid 66 (42,5 mN/m), Polystyrol (43,5 mN/m), PVC (38,4 mN/m), Polyethylen (36,1 mN/m), Polypropylen (29 mN/m) oder Polytetrafluorethan (22,5 mN/m).

Besonders bevorzugt bestehen beide benachbarten Schichten aus einem derartigen unpolaren Polymeren.

Die für die Haftvermittling benötigte Menge des Polypeptids beträgt im allgemeinen 0,01 bis 1000 mg (Milligramm/m² (Quadratmeter), insbesondere 0,01 bis 100 mg/m² und besonders bevorzugt 0,1 bis 10 mg/m².

Das Polypeptid kann auf eine der benachbarten Schichten aufgetragen werden, alternativ kann das Polypeptid auch auf beide Schichten aufgetragen werden, wenn beide Schichten bereits vorgebildet sind.

Das Polypeptid liegt vorzugsweise als wässrige Lösung vor; der Polypeptidgehalt der Lösung beträgt vorzugsweise 0,01 bis 5 Gew.-Teile Polypeptid auf 100 Gew.-Teile Wasser. Für die erfindungsgemäße Verwendung wird die Lösung vorzugsweise weiter verdünnt auf eine Konzentration von 1 bis 10000 µg/ml Wasser, insbesondere 10 bis 1000 µg/ml Wasser.

Nach dem Auftragen kann daher zunächst eine Trocknung zur Entfernung des Wassers erfolgen.

Danach können die beiden benachbarten Schichten durch übliche Methoden, z. B. durch Kaschierung verbunden werden.

Insbesondere kann das Polypeptid auch auf eine der benachbarten Schichten (erste Schicht) aufgetragen werden und die andere benachbarte Schicht) (zweite Schicht) danach durch Aufbringen einer Polymerdispersion, Polymerlösung oder eines lösemittelfreien Polymeren auf die erste, mit dem Haftvermittler versehene Schicht und anschließende Verfilmung und/oder thermische oder photochemische Härtung hergestellt werden. Insbesondere liegt das Polymer dazu in Form einer wässrigen Dispersion oder Lösung vor, besonders bevorzugt handelt es sich um eine wässrige Dispersion eines Emulsionspolymerisats, vorzugsweise eines oben aufgeführten radikalischen Polymeren. Nach dem Auftragen des Polymeren erfolgt dann gegebenenfalls eine Trocknung.

### Zum beschichteten Substrat

Substrate werden zu unterschiedlichsten Zwecken beschichtet. Genannt seien insbesondere dekorative Überzüge oder Schutzüberzüge (zusammenfassend Lackierungen) oder auch Klebstoffbeschichtungen, wobei der Klebstoff als solcher auf das Substrat aufgebracht werden kann oder z. B. in Form eines selbstklebenden Artikels (Etikett oder Klebeband) aufgeklebt wird.

Das Substrat, bzw.die Substratoberfläche, kann aus einem beliebigen Material bestehen. Ebenso kann die Beschichtung bzw.die substratseitige Oberfläche der Beschichtung aus einem beliebigen Material bestehen.

Vorzugsweise besteht die Substratoberfläche oder die Beschichtung, bzw. die substratseitige Oberfläche der Beschichtung aus natürlichen oder synthetischen Polymeren.

Als Polymere kommen insbesondere Polykondensate, wie Polyester, Polyaddukte wie Polyurethane, Polyamide, Polycarbonate oder Polyphenylenether oder Polyphenylensulfide oder Polymere, welche durch radikalische oder ionische Polymerisation von ethylenisch ungesättigten Verbindungen erhältlich sind (kurz radikalische Polymere genannt).

Zum Aufbau der radikalischen Polymeren und ihren Gehalt an Hauptmonomeren gilt das oben gesagte,

Die erfindungsgemäßen Polypeptide eignen sich insbesondere auch als Haftvermittler für unpolare Polymere; daher besteht vorzugsweise zumindest die Substratoberfläche oder die substratseitige Oberfläche der Beschichtung aus einem unpolaren Polymer.

Für den Kontaktwinkel als Maß für die Polarität gilt ebenfalls das oben gesagte.

Besonders bevorzugt bestehen sowohl die Substratoberfläche als auch die substratseitige Oberfläche der Beschichtung aus einem derartigen unpolaren Polymeren.

Die für die Haftvermittling benötigte Menge des Polypeptids entspricht der oben angegebenen Menge.

Das Polypeptid kann auf die Substratoberfläche,auf die substratseitige Oberfläche der Beschichtung oder auf beide aufgetragen werden. Aufbringen auf die substratseitige Oberfläche der Beschichtung ist dann möglich, wenn die Beschichtung vorgebildet ist, d. h. wenn eine einfache Folie oder einen mehrschichtigen Verbund (s.o.) auf das Substrat aufgebracht werden soll.

Das Polypeptid liegt vorzugsweise als wässrige Lösung vor; der Gehalt der Lösung ist wie oben angegeben.

Nach dem Auftragen kann daher zunächst eine Trocknung zur Entfernung des Wassers erfolgen.

Die Beschichtung kann nach üblichen Methoden auf das Substrat aufgebracht werden, Folien oder mehrschichtige Verbunde können z. B. aufkaschiert werden.

Insbesondere kann die Beschichtung durch Aufbringen einer Polymerdispersion, Polymerlösung oder eines lösemittelfreien Polymeren auf die mit dem Haftvermittler versehene subtratseitige Oberfläche und anschließende Verfilmung und/oder thermische oder photochemische Härtung hergestellt werden. Insbesondere liegt das Polymer dazu in Form einer wässrigen Dispersion oder Lösung vor, besonders bevorzugt handelt es sich um eine wässrige Dispersion eines Emulsionspolymerisats, vorzugsweise eines oben aufgeführten radikalischen Polymeren. Nach dem Auftragen des Polymeren erfolgt dann gegebenenfalls eine Trocknung.

Die erfindungsgemäßen mehrschichtigen Verbunde und beschichteten Substrate haben eine deutlich erhöhte Festigkeit. Die Beschichtung haftet durch die Verwendung der Polypeptide als Haftvermitteler stärker auf dem Substrat und die einzelnen Schichten des mehrschichtigen Verbunds haften besser aneinander.

In einer weiteren bevorzugten Ausführungsform der Erfindung handelt es sich bei dem Substrat um ein Metall. Es kann sich hierbei prinzipiell beliebige Metalle handeln. Beispiele umfassen Eisen, Stahl, Zink, Zinn, Aluminium, Kupfer bzw. Legierungen dieser Metalle untereinander sowie mit anderen Metallen handeln. Es kann sich insbesondere um Stahl, mit Zink-, Zinklegierungen, Aluminium- oder Aluminiumlegierungen beschichteten Stahl oder um Aluminium- bzw. Aluminiumlegierungen handeln. Besonders bevorzugt sind Zink- bzw. Zinklegierungen bzw. damit Substrate wie beispielsweise verzinkter Stahl,

Zn- oder Al-Legierungen sind dem Fachmann bekannt. Je nach dem gewünschten Anwendungszweck wählt der Fachmann Art und Menge von Legierungsbestandteilen aus. Typische Bestandteile von Zink-Legierungen umfassen insbesondere Al, Pb, Si, Mg, Sn, Cu oder Cd. Typische Bestandteile von Aluminium-Legierungen umfassen insbesondere Mg, Mn, Si, Zn, Cr, Zr, Cu oder Ti. Es kann sich auch um Al/Zn-Legierungen handeln, bei denen Al- und Zn in annähernd gleicher Menge vorhanden sind. Mit derartigen Legierungen beschichteter Stahl ist kommerziell erhältlich.

Die Metalle können in beliebiger Form vorliegen, bevorzugt sind aber Metallfolien, Metallbänder oder Metallbleche. Bei dem Metall kann es sich auch um ein Verbundmaterial mit einer metallischen Oberfläche handeln. Beispielsweise kann es sich um einen Verbund aus einer Polymerfolie und einem Metall handeln.

Die metallischen Oberflächen werden mit den erfindungsgemäß einzusetzenden Polypeptiden, bevorzugt mit Hydrophobinen als Haftvermittler beschichtet werden. Dies kann mit wässrigen Lösungen der Polypeptiden erfolgen. Einzelheiten zur Beschichtung wurden bereits vorstehend erwähnt.

Bei der Beschichtung kann es sich insbesondere um typische Lacke bzw. Lacksysteme zur Beschichtung von metallischen Oberflächen handeln. Es kann sich hierbei um thermisch, fotochemisch oder nach anderen Mechanismen härtende Lacke handeln.

Typische Lacke zur Beschichtung von Metalloberflächen umfassen mindestens ein Bindemittel sowie vernetzbare Komponenten. Bei den vernetzbaren Komponenten kann es sich um Vernetzer handeln, welche zusätzlich zu einem Bindemittel eingesetzt werden oder es kann sich hierbei um vernetzbare Gruppen handeln, welche mit dem Bindemittel verbunden sind. Selbstverständlich kann auch das Bindemittel vernetzbare Gruppen aufweisen und zusätzlich ein Vernetzer eingesetzt werden. Hierbei sind verschiedene Kombinationsmöglichkeiten denkbar. Beispielsweise können Bindemittel und Vernetzer getrennt voneinander eingesetzt werden. Das Bindemittel umfasst dann reaktive funktionelle Gruppen, die mit komplementären, reaktiven funktionellen Gruppen in den Vernetzern reagieren können. Alternativ kann es sich auch um selbstvernetzende Bindemittel handeln, die reaktive funktionelle Gruppen umfassen, die mit Gruppen ihrer Art ("mit sich selbst") oder mit komplementären, reaktiven funktionellen Gruppen am selben Polymer Vernetzungsreaktionen eingehen können. Möglich ist auch, dass ausschließlich die Vernetzer miteinander reagieren.

Beispiele geeigneter Bindemittel umfassen (Meth)acrylat(co)polymerisate, partiell verseifte Polyvinylester, Polyester, Alkydharze. Polylactone, Polycarbonate, Polyether, Epoxidharz-Amin-Addukte, Polyharnstoffe, Polyamide, Polyimide oder Polyurethane. Selbstverständlich können auch Mischungen verschiedener Polymerer eingesetzt werden, vorausgesetzt es treten durch die Mischung keine unerwünschten Effekte auf.

Die vernetzenden Komponenten können thermisch vernetzende Gruppen oder fotochemisch vernetzende Gruppen aufweisen. Geeignete thermische Vernetzer sind beispielsweise Vernetzer auf Basis von Epoxiden, von Melamin oder von blockierten Isocyanaten. Geeignete Vernetzer zur photochemischen Vernetzung sind insbesondere Verbindungen mit mehreren ethylenisch ungesättigten Gruppen, insbesondere di- oder polyfunktionelle Acrylate.

Durch die erfindungsgemäße Verwendung von Polypeptiden, bevorzugt von Hydrophobinen wird die Haftung des Lackes auf dem Substrat vorteilhaft verbessert. Weiterhin wird in Korrosionsschutztests auch eine verbesserte Beständigkeit gegen Unterwanderung der Lackschicht erreicht.

Die folgenden Beispiele sollen die Erfindung näher illustrieren:

### Teil A) Herstellung von Hydrophobinen

### Beispiel 1

### Vorarbeiten für die Klonierung von yaad-His₆/ yaaE-His₆

Mit Hilfe der Oligonukleotide Hal570 und Hal571 (Hal 572/ Hal 573) wurde eine Polymerase Kettenreaktion durchgeführt. Als Template DNA wurde genomische DNA des Bakteriums Bacillus subtilis verwendet. Das erhaltene PCR Fragment enthielt die codierende Sequenz des Gens yaaD / yaaE aus Bacillus subtilis, und an den Enden je eine Ncol bzw. Bglll Restriktionsschnittstelle. Das PCR Fragment wurde gereinigt und mit den Restriktionsendonukleasen Ncol und BgIII geschnitten. Dieses DNA Fragment wurde als Insert verwendet, und in den zuvor mit den Restriktionsendonukteasen NcoI und BgIII linearisierten Vektor pQE60 der Firma Qiagen kloniert. Die so enstandenen Vektoren pQE60YAAD#2 / pQE60YaaE#5 können zur Expression von Proteinen bestehend aus, YAAa::HIS₆ bzw. YAAE::HIS₆ verwendet werden.
Hal570: gcgcgcccatggctcaaacaggtactga
Hal571: gcagatctccagccgcgttcttgcatac
Hal572: ggccatgggattaacaataggtgtactagg
Hal573: gcagatcttacaagtgccttttgcttatattcc

### Beispiel 2

### Klonierung von yaad-Hydrophobin DewA-HiS₆

Mit Hilfe der Oligonukleotide KaM 416 und KaM 417 wurde eine Polymerase Kettenreaktion durchgeführt. Als Template DNA wurde genomische DNA des Schimmelpilzes Aspergillus nidulans verwendet. Das erhaltene PCR Fragment enthielt die codierende Sequenz des Hydrophobin Gens dewA und einer N-Terminalen FaktorXa Proteinase Schnittstelle. Das PCR Fragment wurde gereinigt und mit der Restriktionsendonuklease BamHI geschnitten. Dieses DNA Fragment wurde als Insert verwendet, und in den zuvor mit der Restriktionsendonuklease BgIII linearisierten Vektor pQE60YAAD#2 kloniert.

Der so enstandene Vektor #508 kann zur Expressions eines Fusionsproteins bestehend aus, YAAD::Xa::dewA::HIS₆ verwendet werden.

### KaM416: GCAGCCCATCAGGGATCCCTCAGCCTTGGTACCAGCGC

KaM417: CCCGTAGCTAGTGGATCCATTGAAGGCCGCATGAAGTT-
CTCCGTCTCCGC

### Beispiel 3

Klonierung von yaad-Hydrophobin RodA-His₆

Die Klonierung des Plasmids #513 erfolgte analog zu Plasmid #508 unter Verwendung der Oligonukleotide KaM 434 und KaM 435.

### KaM434: GCTAAGCGGATCCATTGAAGGCCGCATGAAGTTCTCCATTGCTGC KaM435: CCAATGGGGATCCGAGGATGGAGCCAAGGG

### Beispiel 4

### Klonierung von yaad-Hydrophobin BASF1-HiS₆

Die Klonierung des Plasmids #507 erfolgte analog zu Plasmid #508 unter Verwendung der Oligonukleotide KaM 417 und KaM 418. Als Template DNA wurde ein künstlich synthetisierte DNA Sequenz - Hydrophobin BASF1 -eingesetzt. (siehe Anhang).

KaM417:CCCGTAGCTAGTGGATCCATTGAAGGCCGCAT-
GAAGTTCTCCGTCTCCGC

### KaM418: CTGCCATTCAGGGGATCCCATATGGAGGAGGGAGACAG

### Beispiel 5

### Klonierung von yaad-Hydrophobin BASF2-His₆

Die Klonierung des Plasmids #506 erfolgte analog zu Plasmid #508 unter Verwendung der Oligonukleotide KaM 417 und KaM 418. Als Template DNA wurde ein künstlich synthetisierte DNA Sequenz - Hydrophobin BASF2 -eingesetzt (siehe Anhang).

KaM417:CCCGTAGCTAGTGGATCCATTGAAGGCCGCAT-
GAAGTTCTCCGTCTCCGC

### KaM418: CTGCCATTCAGGGGATCCCATATGGAGGAGGGAGACAG

### Beispiel 6

### Klonierung von yaad-Hydrophobin SC3-His₆

Die Klonierung des Plasmids #526 erfolgte analog zu Plasmid #508 unter Verwendung der Oligonukleotide KaM464 und KaM465. Als Template DNA wurde cDNA von Schyzophyllum commune eingesetzt (siehe Anhang).
KaM464: CGTTAAGGATCCGAGGATGTTGATGGGGGTGC
KaM465: GCTAACAGATCTATGTTCGCCCGTCTCCCCGTCGT

### Beispiel 7

### Fermentation des rekombinanten E.coli Stammes yaad-Hydrophobin DewA-His₆

Inokulation von 3ml LB Flüssigmedium mit einem yaad-Hydrophobin DewA-His₆ exprimierenden E.coli Stamm in 15ml Greiner Röhrchen. Inkubation für 8h bei 37°C auf einem Schüttler mit 200 UpM. Je 21l Erlenmeyer Kolben mit Schikanen und 250ml LB Medium (+ 100µg/ml Ampicillin) werden mit jeweils 1ml der Vorkultur angeimpft und 9h bei 37°C auf einem Schüttler mit 180 UpM inkubiert. 13.51 LB-Medium (+100µg/ml Ampicillin) in einem 20I Fermenter mit 0,51 Vorkultur (OD₆₀₀ₙₘ 1:10 gegen H₂O gemessen) animpfen. Bei einer OD₆₀ₙₘ von ∼3.5 Zugabe von 140ml 100mM IPTG. Nach 3h Fermenter auf 10°C abkühlen und Fermentationsbrühe abzentrifugieren. Zellpellet zur weiteren Aufreinigung verwenden.

### Beispiel 8

Reinigung des rekombinanten Hydrohobin-Fusionsproteins (Reinigung von Hydrophobin-Fusionsproteinen, die ein C-terminales His6-tag besitzen)

100 g Zellpellet (100-500 mg Hydrophobin) werden mit 50 mM Netriumphasphatpuffer. pH 7,5 auf 200 ml Gesamtvolumen aufgefüllt und resuspendiert. Die Suspension wird mit einem Ultraturrax Typ T25 (Janke und Kunkel; IKA-Labortechnik) für 10 Minuten behandelt und anschliessend für 1 Stunde bei Raumtemperatur mit 500 Einheiten Benzonase (Merck, Darmstadt; Best -Nr 1.01697.0001) zum Abbau der Nukleinsäuren inkubiert. Vor dem Zellaufschluss wird mit einer Glaskartusche (P1) filtriert. Zum Zellaufschluß und für das Scheren der restlichen genomischen DNA werden zwei Homogenisatorläufe bei 1.500 bar durchgeführt (Microfluidizer M-110EH; Microfluidics Corp.). Das Homogenisat wird zentrifugiert (Sorvall RC-5B, GSA-Rotor, 250 ml Zentrifugenbecher. 60 Minuten, 4°C, 12.000 Upm, 23.000 g), der Überstand auf Eis gestellt und das Pellet in 100 ml Natriumphosphatpuffer, pH 7,5 resuspendiert. Zentrifugation und Resuspendieren werden dreimal wiederholt, wobei der Natriumphosphatpuffer bei der dritten Wiederholung 1 % SDS enthält. Nach der Resuspension wird für eine Stunde gerührt und eine abschliessende Zentrifugation durchgeführt (Sorvall RC-5B. GSA-Rotor, 250 ml Zentrifugenbecher, 60 Minuten, 4°C. 12.000 Upm, 23.000 g). Gemäß SDS-PAGE Analyse ist das Hydrophobin nach der abschliessenden Zentrifugation im Überstand enthalten (Abbildung 1). Die Versuche zeigen, dass das Hydrophobin wahrscheinlich in Form von Einschlusskörpern in den entsprechenden E.coli Zellen enthalten ist. 50 ml des Hydrophobin-enthaltenden Überstandes werden auf eine 50 ml Nickel-Sepharose High Performance 17-5268-02 Säule aufgetragen (Amersham), die mit 50 mM Tris-Cl pH 8,0 Puffer äquilibriert wurde. Die Säule wird mit 50 mM Tris-Cl pH 8,0 Puffer gewaschen und das Hydrophobin anschliessend mit 50 mM Tris-Cl pH 8,0 Puffer, der 200 mM Imidazol enthält, eluiert. Zur Entfernung des Imidazols wird die Lösung gegen 50 mM Tris-Cl pH 8,0 Puffer dialysiert.

Abbildung 1 zeigt die Reinigung des hergestellten Hydrophobins:
Spur 1: Auftrag Nickel-Sepharose Säule (1:10 Verdünnung)
Spur 2: Durchlauf = Eluat Waschschritt
Spuren 3 - 5: OD 280 Maxima der Elutionsfraktionen

Das Hydrophobin der Abbildung 1 besitzt ein Molekulargewicht von ca. 53 kD. Die kleineren Banden repräsentieren zum Teil Abbauprodukte des Hydrophobins.

### Beispiel 9

Anwendungstechnische Prüfung; Charakterisierung des Hydrophobins durch Kontaktwinkeländerung eines Wassertropfens auf Glas

### Substrat:

Glas (Fensterglas, Süddeutsche Glas, Mannheim): Konzentration Hydrophobin: 100 µg/mL
Inkubation von Glasplättchen über Nacht (Temperatur 80°C) in 50mM Na-Acetat pH 4 + 0,1% Tween 20
danach Beschichtung waschen in destilliertem Wasser
danach Inkubation 10min / 80°C / 1% SDS-Lösung in dest Wasser Waschen in dest. Wasser

Die Proben werden an der Luft getrocknet und der Kontaktwinkel (in Grad) eines Tropfens von 5 µl Wasser bestimmt.

Die Kontaktwinkelmessung wurde auf einem Gerät Dataphysics Contact Angle System OCA 15+, Software SCA 20.2.0. (November 2002) bestimmt. Die Messung erfolgte gemäss den Herstellerangaben.

Unbehandeltes Glas ergab einen Kontaktwinkel von 30 ± 5°; eine Beschichtung mit dem funktionellen Hydrophobin gemäss Beispiel 8 (yaad-dewA-his₆) ergab Kontaktwinkel von 75 ± 5°.

### Teil B) Verwendung von Polypeptiden als Haftvermittler

### Beispiel 10 (nicht erfindungsgemäß)

### Substrat aus Polyethylen

Verwendete Materialien:
Verwendete Lösung:
   Für die anwendungstechnischen Versuche wurde eine Lösung des gemäß Beispiel 8 hergestellten Fusionsproteines yaad-Xa-dewA-his (SEQ ID NO: 19) in Wasser eingesetzt. Konzentration des Hydrophobins in Lösung: 100 µg/ml (0,01 Gew.-%).
Substrat: Formkörper (Plättchen) aus Polyethylen
Beschichtung:
   Eine Polyesterfolie wurde mit Acronal A 240, eine handelsübliche, wässsrige Polyacrylatdispersion der BASF für Haftklebstoffe, beschichtet und getrocknet und in 2,5 cm breite Streifen geschnitten. Die erhaltenen Klebstreifen wurden zur Beschichtung der PE Plättchen verwendet.
Durchführung:
   Die Polypeptid-Lösung wurde auf Polyethylen-Plättchen aufgetragen und getrocknet (vorbehandelte Polyethylen-Plättchen).

Anschließend wurden Klebestreifen auf vorbehandelte und zum Vergleich auf nicht vorbehandelte Polyethylen-Plättchen geklebt und die zum Abziehen der Klebestreifen notwendige Kraft bestimmt (Schälfestigkeit in N)
Schälfestigkeit mit Polypeptid als Haftvermittler: 4,7 N
Schälfestigkeit ohne Polypeptid als Haftvermittler: 2,6 N

### Beispiel 11

### Metallische Substrate

Für die anwendungstechnischen Versuche wurde eine Lösung des gemäß Beispiel 8 hergestellten Fusionsproteines yaad-Xa-dewA-his (SEQ ID NO: 19) in Wasser eingesetzt. Konzentration des Hydrophobins in Lösung: 100 µg/ml (0,01 Gew.-%). Gibt es Angaben zum pH-Wert? Wenn kein Puffer zusätzlich zugesetzt wurde, müsste das 8 bis 8,5 sein?

Als metallische Substrate wurden die folgenden Prüfbleche eingesetzt:

| Nr. | Substrat |
|---|---|
| Beispiel 11-1 | Stahl (Typ ST 2 (Werkstoffnummer 1.0330)) |
| Beispiel 11-2 | Verzinkter Stahl (Sendzimirverzinkte Stahlbleche GARDOBOND OE HDG/2 (Fa. Chemetall) |
| Beispiel 11-3 | Aluminium (AlMgSi AA 6016 GARDOBOND Unbehandelt (Fa. Chemetall)) |

Als Lack wurde ein Einbrenndecklack auf Alkyd-Melaminbasis eingesetzt. (Reicht das, um den Lack zu charakterisieren?)

### Versuchsbeschreibung

Die Aluminiumbleche wurden in einem alkalischen Tauchreinigungsbad (60 g/l NaOH, 60°C, 1 min) gebeizt und mit dest. Wasser gespült. Anschließend wurden die Bleche in einem sauren Dekapierbad mit HNO₃/H2O (1:1) bei Raumtemperatur für 15 Sekunden dekapiert, mit dest. Wasser gespült und mit Druckluft trocken geblasen.

Die verzinkten Stahlbleche und die Stahlbleche wurden mit heißem Flusswasser gespült, mit destilliertem Wasser nachgespült und mit Druckluft trocken geblasen.

Zur Beschichtung mit den Hydrophobinen wurden die Bleche jeweils bei Raumtemperatur in die oben genannte Lösung eingetauscht. Die Aluminiumbleche wurden 16 h, die verzinkten Stahlbleche und die Stahlbleche 4 h eingetaucht. Anschließend wurden die Bleche mit dest. Wasser gespült und mit Druckluft trocken geblasen.

Nach dem Beschichten mit dem Hydrophobin als Haftvermittler wurden die Bleche in einer Kunststoffschale für 15 s in einen Einbrenndecklack auf Alkyd-Melaminbasis getaucht und an der Luft ca. 1 h vorgetrocknet. Anschließend wurde im Trockenschrank bei 190°C für 30 min getrocknet und ausgehärtet.

Zu Vergleichszwecken wurde jeweils eine weitere Probe in gleicher Art und Weise mit dem Lack, aber ohne den Hydrophobin-Haftvermittler präpariert.

### Anwendungstechnische Prüfung

Die anwendungstechnische Beurteilung der Bleche erfolgte nach EN ISO 2409 (Gitterschnitt), EN ISO 4628-8 (Unterwanderung) und DIN 53156 (Erichsen-Tiefung).

Beim Gitterschnitttest wird das Aussehen der Lackoberfläche nach dem Einschneiden eines Gitters in die Oberfläche anhand von vorgegebenen Standards bewertet. Bewertet wird hierbei, in welchem Maße der Lack durch das Einschneiden des Gitters von der Oberfläche abplatzt. Die Bewertung erfolgt in bekannter Art und Weise mit Hilfe von Noten 0 bis 5, wobei 0 für die beste und 5 für die schlechteste Bewertung steht.

Die Unterwanderung der Lackschicht wird nach einem Standard-Korrosionstest (Belastung in der Salzsprühkammer (SS DIN 50021)) mit den Blechen ermittelt. Die Unterwanderung der Aluminiumbleche wurde nach 298 h, die der Stahlbleche nach 50 h und die der verzinkten Stahlbleche nach 190 h anhand von vorgegebenen Standards mit den Noten 0 bis 5 bewertet, wobei 0 für die beste und 5 für die schlechteste Bewertung steht.

Bei der Erichsen-Tiefung wird eine Kugel von der Rückseite gegen die Probe gedrückt und eine Vertiefung eingedrückt. Bewertet wird das Aussehen des Lackes an der Druckstelle anhand von vorgegeben Standards, wobei in diesem Falle 5 für die beste und 0 für die schlechteste Bewertung steht.

Die Ergebnisse der anwendungstechnischen Prüfungen sind im Folgenden zusammengefasst.

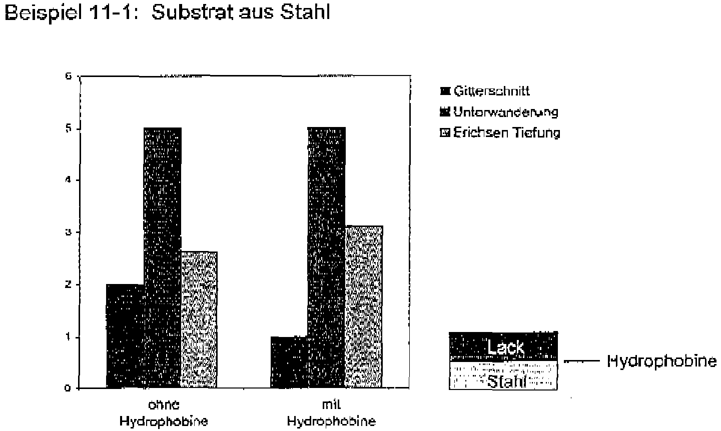

Durch die Verwendung eines Hydrophobins als Haftvermittler bleibt die Unterwanderung mit Note 5 gegenüber einer Probe ohne Hydrophobin unverändert. Das Ergebnis des Gitterschnitttests (kleinerer Wert) und des Erichsen-Tiefungs-est (größerer Wert) fällt jeweils besser aus.

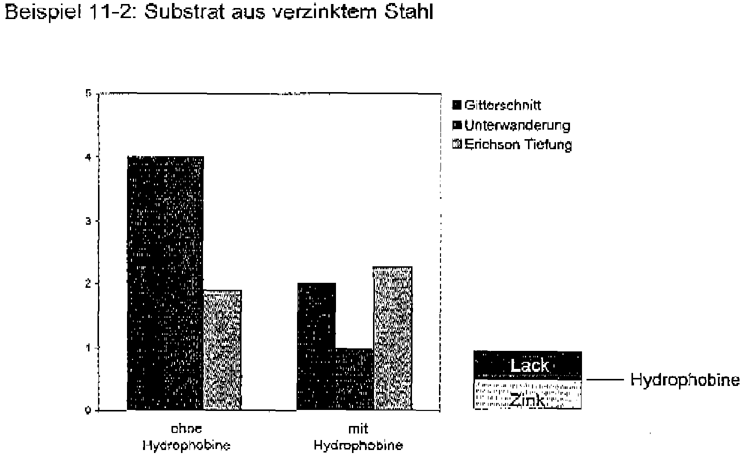

Auf verzinktem Stahl werden bei Verwednung von Hydrophobinen als Haftvermittler beiallen drei Werten deutlich bessere Werte erzielt, als ohne Haftvermittler (kleinere Werte bei Gitterschnitt und Unterwanderung und größerer Wert bei Erichsen-Tiefung). Die deutlichste Verbesserung wird bei der Korrosionsschutzbeständigkeit (Unterwanderung) erzielt (Note 1 mit Haftvermittler im Gegensatz zu Note 4 ohne Haftvermittler).

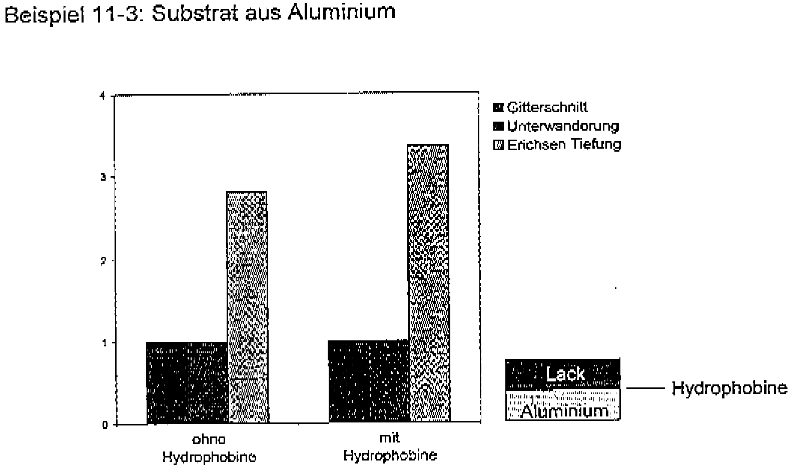

Bei einem Aluminim-Substrat sind Gitterschnitt und Unterwanderung schon ohne Haftvermittler gut. Bei der Erichsen-Tierfung ergibt sich noch eine leichte Verbesserung.

## Patentansprüche

1. Beschichtetes metallisches Substrat enthaltend als Haftungsvermittler zwischen der Beschichtung und dem Substrat Verbindungen, welche zu mindestens 40 Gew.-% aus über Peptidbindungen verknüpften alpha-Aminosäuren aufgebaut sind (im nachfolgenden kurz Polypeptid genannt), **dadurch gekennzeichnet, dass** es sich bei den Polypeptiden um Hydrophobine der Formel
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)
handelt, wobei X jeweils gleich oder verschieden sein kann und für jede der 20 natürlich vorkommenden Aminosäuren (Phe, Leu, Ser, Tyr, Cys, Trp, Pro, His, Gln, Arg, Ile Met, Thr, Asn, Lys, Val, Ala, Asp, Glu, Gly) stehen kann, die bei X stehenden Indizes jeweils die Anzahl der Aminosäuren darstellen, und C für Cystein, Alanin, Serin, Glycin, Methionin oder Threonin steht, wobei mindestens vier der mit C benannten Reste für Cystein stehen, und weiterhin die Indizes n und m unabhängig voneinander für natürliche Zahlen von 0 und 500, bevorzugt von 15 bis 300 stehen, und wobei die Menge der Hydrophobine 0,01 bis 100 mg/m² beträgt.

2. Beschichtetes metallisches Substrat gemäß Anspruch 1, **dadurch gekennzeichnet, dass** es sich bei dem Substrat um eines ausgewählt aus der Gruppe von Stahl, mit Zink-, Zinklegierungen, Aluminium- oder Aluminiumlegierungen beschichteten Stahl oder um Aluminium- bzw. Aluminiumlegierungen handelt.

3. Beschichtetes metallisches Substrat gemäß Anspruch 1, **dadurch gekennzeichnet, dass** es sich bei dem Substrat um verzinkten Stahl handelt.

4. Beschichtetes metallisches Substrat gemäß einem der Ansprüche 1 bis 3, **dadurch gekennzeichnet, dass** es sich bei der Beschichtung um eine Lackschicht handelt.

5. Beschichtetes metallisches Substrat gemäß einem der Ansprüche 1 bis 4, **dadurch gekennzeichnet, dass** die Menge der Hydrophobine 0,01 bis 10 mg/m² beträgt.

6. Verfahren zur Herstellung des beschichteten metallischen Substrats gemäß einem der Ansprüche 1 bis 5 **dadurch gekennzeichnet, dass**
- das Hydrophobin als wässrige Lösung in einer Konzentration von 1 bis 1000 µg/ml auf die Substratoberfläche aufgebracht wird,
- eine Trocknung erfolgt, und
- anschließend das Beschichtungsmittel auf das beschichtete Substrat aufgebracht wird.

7. Verfahren gemäß Anspruch 6, **dadurch gekennzeichnet, dass** es sich bei dem Beschichtungsmittel um einen Lack handelt.

## Claims

1. A coated metallic substrate comprising compounds, at least 40% by weight of which are composed of alpha-amino acids linked via peptide bonds (referred to as polypeptide for short hereinbelow), as adhesion promoters between the coating and the substrate, wherein the polypeptides are hydrophobins of the formula
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I),
where each X may be identical or different and may be any of the 20 naturally occurring amino acids (Phe, Leu, Ser, Tyr, Cys, Trp, Pro, His, Gln, Arg, Ile Met, Thr, Asn, Lys, Val, Ala, Asp, Glu, Gly), the indices next to X indicate in each case the number of amino acids, and C is cysteine, alanine, serine, glycine, methionine or threonine, with at least four of the residues denoted C being cysteine, and furthermore the indices n and m being independently of one another natural numbers from 0 and 500, preferably from 15 to 300, and the amount of hydrophobins being from 0.01 to 100 mg/m².

2. The coated metallic substrate according to claim 1, wherein the substrate is one selected from the group consisting of steel, steel coated with zinc, zinc alloys, aluminum or aluminum alloys, or is aluminum or aluminum alloys.

3. The coated metallic substrate according to claim 1, wherein the substrate is galvanized steel.

4. The coated metallic substrate according to any of claims 1 to 3, wherein the coating is a paint layer.

5. The coated metallic substrate according to any of claims 1 to 4, wherein the amount of hydrophobins is from 0.01 to 10 mg/m².

6. A process for preparing the coated metallic substrate according to any of claims 1 to 5, wherein
- the hydrophobin is applied as an aqueous solution at a concentration of from 1 to 1000 µg/ml to the substrate surface,
- a drying procedure is carried out, and
- the coating agent is then applied to the coated substrate.

7. The process according to claim 6, wherein the coating agent is a paint.

## Revendications

1. Substrat métallique revêtu, contenant en tant que promoteur d'adhérence entre le revêtement et le substrat des composés qui sont constitués à raison d'au moins 40 % en poids d'acides alpha-aminés liés par des liaisons peptidiques (ci-après appelés en abrégé polypeptides), **caractérisé en ce que**, pour ce qui concerne les polypeptides, il s'agit d'hydrophobines de formule
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)
dans laquelle chaque X peut être identique ou différent et peut représenter chacun des 20 acides aminés naturels (Phe, Leu, Ser, Tyr, Cys, Trp, Pro, His, Gln, Arg, Ile, Met, Thr, Asn, Lys, Val, Ala, Asp, Glu, Gly), les indices représentés par X indiquent le nombre des différents acides aminés, et C représente la cystéine, l'alanine, la sérine, la glycine, la méthionine ou la thréonine, au moins quatre des résidus appelés C représentent la cystéine, et en outre les indices n et m représentent, indépendamment les uns des autres, des nombres entiers de 0 et 500, de préférence de 15 à 300, et la quantité des hydrophobines est de 0,01 à 100 mg/m².

2. Substrat métallique revêtu selon la revendication 1, **caractérisé en ce que**, pour ce qui concerne le substrat, il s'agit d'un substrat choisi dans le groupe de l'acier, de l'acier revêtu de zinc, d'alliages de zinc, d'aluminium ou d'alliages d'aluminium, ou d'aluminium ou d'alliages d'aluminium.

3. Substrat métallique revêtu selon la revendication 1, **caractérisé en ce que**, en ce qui concerne le substrat, il s'agit d'acier galvanisé.

4. Substrat métallique revêtu selon l'une des revendications 1 à 3, **caractérisé en ce que**, pour ce qui concerne le revêtement, il s'agit d'une couche de vernis.

5. Substrat métallique revêtu selon l'une des revendications 1 à 4, **caractérisé en ce que** la quantité des hydrophobines est de 0,01 à 10 mg/m².

6. Procédé de fabrication du substrat métallique revêtu selon l'une des revendications 1 à 5, **caractérisé en ce que**
- on applique sur la surface du substrat l'hydrophobine sous forme d'une solution aqueuse à une concentration de 1 à 1000 µg/ml,
- on procède à un séchage, et
- puis on applique sur le substrat revêtu la composition de revêtement.

7. Procédé selon la revendication 6, **caractérisé en ce que**, pour ce qui concerne la composition de revêtement, il s'agit d'un vernis.
